# EUROPÄISCHE PATENTSCHRIFT

(11) **EP 2 380 627 B1**
(45) Veröffentlichungstag und Bekanntmachung des Hinweises auf die Patenterteilung: **28.10.2015**
(21) Anmeldenummer: 11160807.1
(22) Anmeldetag: 01.04.2011
(51) Int. Cl.: A61N 1/372

(54) **Sicherheitssystem mit einem Steuergerät für die Kommunikation mit einem medizinischen Implantat und einer Maschine**
Safety system with a control device for communication with a medical implant and a machine
Système de sûreté avec un appareil de commande pour la communication avec un implant médical et une machine

(30) Priorität: 20.04.2010 US 325826 P
(43) Veröffentlichungstag der Anmeldung: 26.10.2011
(73) Patentinhaber: BIOTRONIK SE & Co. KG, 12359 Berlin (DE)
(72) Erfinder: Dörr, Thomas, 12437 Berlin (DE); Diebold, Michael, 12169 Berlin (DE)
(74) Vertreter: Galander, Marcus

(56) Entgegenhaltungen:
- EP-A2- 2 026 229
- WO-A1-2004/024230
- US-A1- 2002 099 424
- US-A1- 2002 169 482
- US-A1- 2007 135 855

## Beschreibung

Die Erfindung betrifft ein Sicherheitssystem nach Anspruch 1. Das Sicherheitssystem umfasst ein Steuergerät, welches einerseits Daten von einem medizinischen Implantat empfangen kann und andererseits Daten, die mit den empfangenen Daten im Zusammenhang stehen, an eine Maschinensteuerung weitergeben kann, um das Verhalten der Maschine und insbesondere deren Reaktion auf Bedieneingriffe eines Benutzers zu beeinflussen.

Bereits ein solches Steuersystem ist bisher nicht bekannt. Es dient dazu, das beispielsweise aus US 6,480,744 B2 bekannte Problem zu lösen, dass beispielsweise die Fahrtauglichkeit des Trägers eines Implantates wie beispielsweise eines Herzschrittmachers unter bestimmten, vom Implantat zu erfassenden Bedingungen eingeschränkt sein kann und es daher wünschenswert ist, dass sich ein Fahrzeug als von einem Implantatsträger mit eingeschränkter Fahrtauglichkeit bediente Maschine je nach Grad der Einschränkung der Fahrtauglichkeit automatisch anders verhält. So ist in US 6,480,744 beispielsweise vorgeschlagen, dass eine Fahrzeugsteuerung als Maschinensteuerung ein Fahrzeug abbremst oder stoppt, bevor oder während das medizinische Implantat eine Therapie, beispielsweise einen Defibrillationsschock abgibt. Hierzu hat das Implantat im Vorfeld beispielsweise eine behandlungsbedürftige Defibrillation des Herzens des Implantatsträgers erfasst. In US 6,480,744 ist die unmittelbare Beeinflussung der Fahrzeugsteuerung durch das medizinische Implantat beschrieben.

Ein weiteres Beispiel für die Beeinflussung einer Fahrzeugsteuerung durch ein Implantat ist in US 2002/0099424 A1 beschrieben.

Das erfindungsgemäße Sicherheitssystem unterbindet eine derartige unmittelbare Beeinflussung der Fahrzeugsteuerung als Maschinensteuerung, indem das Steuergerät als Relaisstation zwischen ein Implantat und eine jeweilige Maschinensteuerung geschaltet ist und mit beiden drahtlos kommunizieren kann, so dass die Verbindung zwischen Implantat und Maschinen- bzw. Fahrzeugsteuerung mittelbar ist.

Erfindungsgemäß weist das Steuergerät hierzu eine erste Schnittstelle für eine drahtlose Kommunikation mit einem Implantat und eine zweite Schnittstelle für eine Kommunikation mit einer Maschinensteuerung sowie einen Speicher für eine eindeutige Kennung auf. Die erste Schnittstelle ist ausgebildet, von einem Implantat physiologische oder Daten oder einen Tauglichkeitsindex zu empfangen, der die Tauglichkeit eines Implantatträgers, eine Maschine zu bedienen, beschreibt. Die zweite Schnittstelle ist ausgebildet, den Tauglichkeitsindex zusammen mit der eindeutigen Kennung an eine jeweilige Maschinensteuerung einer jeweiligen Maschine zu übertragen.

Das Steuergerät dient damit einerseits als eine Art Schlüssel, deren Bestandteil die eindeutige Kennung ist, die zum Vollständigen oder teilweisen Aktivieren der Maschinensteuerung erforderlich ist. Zum anderen dient das Steuergerät als Relaisstation zur Übertragung eines Tauglichkeitsindexes an die Maschinensteuerung, wobei dieser Tauglichkeitsindex aus vom Implantat erfassten, physiologische Parameter oder daraus abgeleitete physiologische Zustände beschreibenden physiologische Daten abgeleitet ist. Die physiologischen Daten werden zu diesem Zweck mittels einer Klassifikationseinheit klassifiziert und unterschiedlichen Tauglichkeitsgraden zugeordnet. Der Tauglichkeitsindex beschreibt einen jeweiligen Tauglichkeitsgrad. Die Klassifikationseinheit kann dabei beispielsweise Bestandteil des Steuergeräts sein, alternativ jedoch auch Bestandteil des Implantats. Im ersten Fall sendet das Implantat physiologische Daten an das Steuergerät und diese physiologischen Daten werden durch die Klassifikationseinheit in dem Steuergerät unterschiedlichen Tauglichkeitsgraden zugeordnet und so ein entsprechender Tauglichkeitsindex generiert. Alternativ kann die Zuordnung von physiologischen Daten zu Tauglichkeitsgraden und das Generieren entsprechender Tauglichkeitsindizes auch im Implantat selbst erfolgen. Dann überträgt bereits das Implantat einen jeweiligen Tauglichkeitsindex an das Steuergerät und das Steuergerät reicht diesen Tauglichkeitsindex als Relaisstation zusammen mit der eindeutigen Kennung an eine jeweilige Maschinensteuerung weiter.

Der Vorteil, der mit dem erfindungsgemäßen Steuergerät erreicht wird besteht darin, das anders als beim Stand der Technik das medizinische Implantat und eine jeweilige, vom Implantatträger zu bedienende Maschine, beispielsweise ein Fahrzeug, nicht unmittelbar miteinander kommunizieren müssen und können. Dies erlaubt es, beispielsweise das Steuergerät als Schlüssel losgelöst von einem Implantat zu verwenden. In diesem Falle überträgt das Steuergerät lediglich die eindeutige Kennung und die Maschine kann sich verhalten, wie beispielsweise ein übliches Fahrzeug, dessen Fahrzeugsteuerung als Maschinensteuerung sich wie übliche Fahrzeugsteuerungen verhält, die nicht dazu ausgebildet sind, auf unterschiedliche Fahrtauglichkeitsgrade jeweils anders zu reagieren. Andererseits ist durch die Zuordnung des Steuergeräts zu einem Implantat einerseits und zu einer Maschine, insbesondere einem Fahrzeug andererseits leicht sicher zu stellen, dass ein jeweiliger Implantatträger auch tatsächlich Fahrer eines entsprechenden Fahrzeugs ist (also Bediener einer entsprechenden Maschine) und nicht etwa Beifahrer ist, solange nur der Implantatträger selbst sein jeweils eigenes Steuergerät als Fahrzeugschlüssel benutzt.

In einer ersten besonders bevorzugten Ausführungsvariante ist das Steuergerät als Maschinenschlüssel, insbesondere als Fahrzeugschlüssel, ausgebildet und besitzt hierzu einen entsprechenden Transponder zum Übertragen der eindeutigen Kennung. Die zweite, maschinenseitige drahtlose Schnittstelle ist ausgebildet, im MEDS- (Medical Data Service bei 401-402 MHz und 405-406 MHz) und ISM (Industiral, Scientivic and Medical, bei 433 MHz und 2,4 GHz)-Frequenzbereich zu arbeiten.

Die erste drahtlose Schnittstelle besitzt vorzugsweise einen Empfänger, der im MICS-Frequenzbereich arbeitet. Dieser Frequenzbereich liegt im Frequenzband zwischen 402 und 405 MHz und ist für die drahtlose Kommunikation mit medizinischen Implantaten reserviert (MICS: Medical Implant Communication Service).

Erfindungsgemäß umfasst das Sicherheitssystem ein Implantat, ein Steuergerät der zuvor beschriebenen Art und eine Maschine, von denen die Maschine eine Steuereinheit aufweist, die ausgebildet ist, einen Betrieb der Maschine nur im Falle des Empfangs einer eindeutigen Kennung zu ermöglichen und eine Betriebsart der Maschine in Abhängigkeit von empfangenen Tauglichkeitsindex einzustellen.

Vorzugsweise ist das Implantat ein Herzstimulator wie z.B. ein Herzschrittmacher oder Kardioverter/Defibrillator. Alternativ kann das Implantat auch ein reines Monitoring-Implantat ohne eigene Therapiefunktion sein.

Die Erfindung soll nun anhand von Ausführungsbeispielen mit Bezug auf die Figuren näher erläutert werden. Von den Figuren zeigt:
- Figur 1: Eine Illustration des Standes der Technik;
- Figur 2: eine Darstellung einer erfindungsgemäßen sicheren Implantat-FahrzeugKommunikation;
- Figur 3: ein erfindungsgemäßes Steuergerät als rückwirkungsfreie Relaisstation;
- Figur 4: eine weitere Illustration zum Stand der Technik;
- Figur 5: eine Illustration zum Ablauf einer Fahrtauglichkeitsklassifikation;
- Figur 6: ein Blockschaltbild eines erfindungsgemäßen Sicherheitssystems;
- Figur 7: einer erste Beispielklassifikation A; und
- Figur 8: eine zu Figur 7 alternative, zweite Beispielklassifikation B

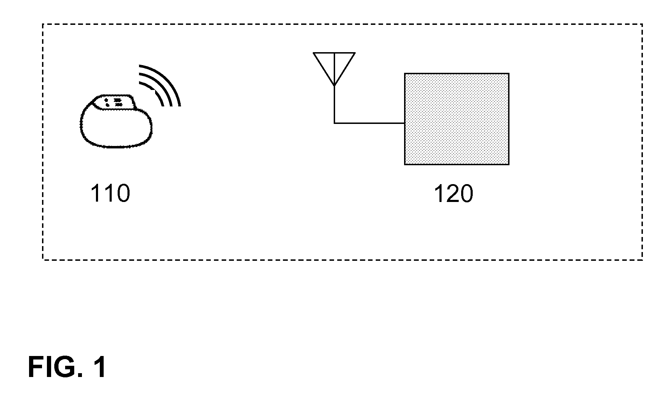

In der Figur 1 ist der in oben genannten Patenten beschriebene Stand der Technik dargestellt. Hier erfolgt eine direkte Kommunikation des Implantates mit den Komponenten des Fahrzeugsystems. Ein elektronisches Implantat 110 enthält einen Sender und ein Fahrzeug 120 enthält einen Empfänger. In diesem System wird nicht unterschieden, ob der Träger des Implantats 110 als Fahrer oder Mitfahrer im Fahrzeug bewegt wird. Dadurch wäre es möglich, dass es zu ungewollten bzw. fehlerhaften Beeinflussungen der Fahrassistenzsysteme durch das Implantat eines Mitfahrers kommen kann. Außerdem muss das gesamte Fahrzeug auch die Anforderungen an ein Medizinproduktesystem hinsichtlich Entwicklung, Zulassung und Produktlebenszyklus erfüllen. Ein rückwirkungsfreier Betrieb des elektronischen Implantates im Fehlerfall des Fahrzeugsystems ist nur extrem schwer zu erbringen.

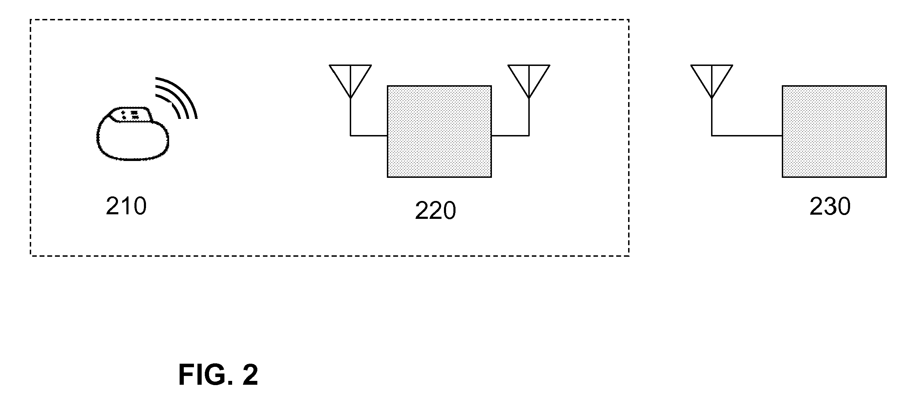

In der Figur 2 ist die erfindungsgemäße Implementierung einer sicheren Implantat-Fahrzeugkommunikation dargestellt. Das elektronische Implantat 210 kommuniziert hier bidirektional in einem für die Implantatkommunikation bevorzugten Band (MICS oder ISM) mit einem Steuergerät 220, das gleichzeitig einen Fahrzeugschlüssel bildet. Der Fahrzeugschlüssel 220 fungiert gleichzeitig immer dann als Relaisstation zu einem Fahrzeug 230, wenn das Fahrzeug mit diesem Schlüssel 220 gestartet wurde. Der Fahrzeugschlüssel 220 (also das Steuergerät) beinhaltet als Relaisstation für die Kommunikation mit dem Implantat 210 die erforderlichen Sicherheitseinrichtungen, z.B. eine Firewall, um eine von Fehlfunktionen des Fahrzeugs rückwirkungsfreien Betrieb des Implantates sicher zu stellen. Die Kommunikation zwischen Fahrzeugschlüssel 220 und Fahrzeug 230 findet auf einem zweiten, von der Implantatkommunikation grundsätzlich unabhängigen Kommunikationskanal statt.

In dieser erfindungsgemäßen Implementierung müssen nur das elektronische Implantat und die Relaisstation als Medizinprodukte klassifiziert werden.

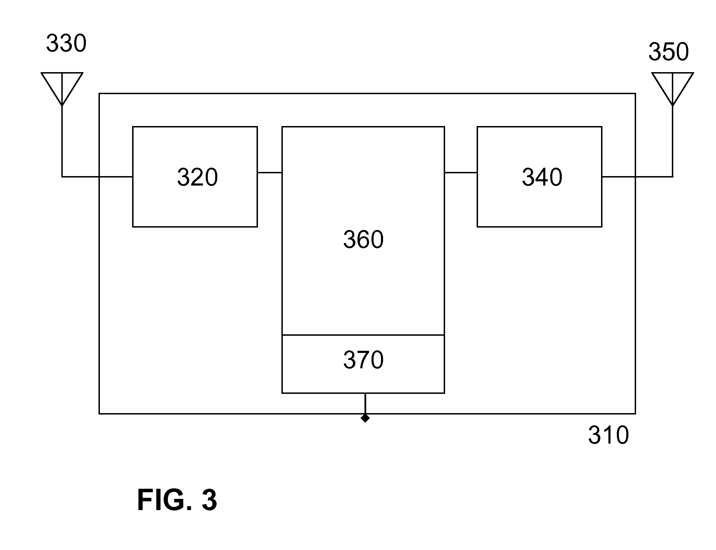

In der Figur 3 ist eine Realisierung der rückwirkungsfreien Kommunikation mit dem Fahrzeug dargestellt. Das hier dargestellte Steuergerät 310 ist als Relaisstation ausgebildet und verfügt über ein erstes Kommunikationsmodul 320, das z.B. im MICS-Band arbeitet und mit einer ersten Antenne 330 verbunden ist. Diese Kommunikationseinheit dient ausschließlich der Kommunikation zwischen elektronischem Implantat und Relaisstation.

Ferner umfasst das Steuergerät 310 ein zweites Kommunikationsmodul 340, das z.B. im ISM- oder MEDS-Band arbeitet und mit einer entsprechenden Antenne 350 für die Kommunikation mit der Fahrzeugelektronik konfiguriert ist. Das ISM-Band wird in Fahrzeug derzeit bereits zur Zugangskontrolle (Keyless Go) eingesetzt.

Über einen Sicherheitsserver 360 wird die Unabhängigkeit der Implantatkommunikation von der Fahrzeugkommunikation gewährleistet. Dieser Sicherheitsserver 360 kann als einfacher Kommandointerpreter oder aber als relativ komplexe Firewall ausgebildet sein und besitzt eine Schnittstelle 370, die eine Wartung des Sicherheitsservers im Medizinproduktesystem erlaubt.

Die so dargestellte Relaisstation kann entweder als ein dem Patienten zugeordnetes mobiles Gerät oder aber als ein im Fahrzeug installiertes System realisiert werden. In der Ausführung im Fahrzeug ist diese Einheit so angebracht, dass diese zu Wartungszwecken durch den Medizinproduktehersteller einfach entfernt werden kann, so dass die Wartungsschnittstelle 370 zugänglich ist. Für die Fahrzeugwartung wiederum ist die Wartungsschnittstelle 370 nicht zugänglich, so dass eine Rückwirkung durch Maßnahmen der Fahrzeugwartung grundsätzlich ausgeschlossen ist.

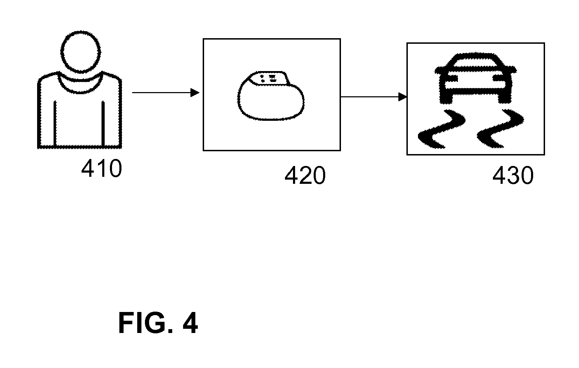

In der Figur 4 ist noch einmal der Stand der Technik dargestellt (vergleiche Figur 1). Ein elektronisches Implantat 420 leitet von physiologischen Parametern des Patienten eine Notwendigkeit ab, einen direkten Eingriff in den Fahrablauf eines Fahrzeuges 430 durchzuführen. Der Eingriff selbst ist hier immer vorherbestimmt (z.B. Bis zum Stillstand abbremsen) und kann optional auch eine Rückkopplung zur Therapiesteuerung des Implantates beinhalten. So ist z.B. die Verzögerung einer Kardioversionstherapie bis zum Stillstand des Fahrzeuges vorgesehen. Keine der bekannten Lösungen sieht eine patientenindividuelle und fahrsituationsabhängige Fahrzeugbeeinflussung vor.

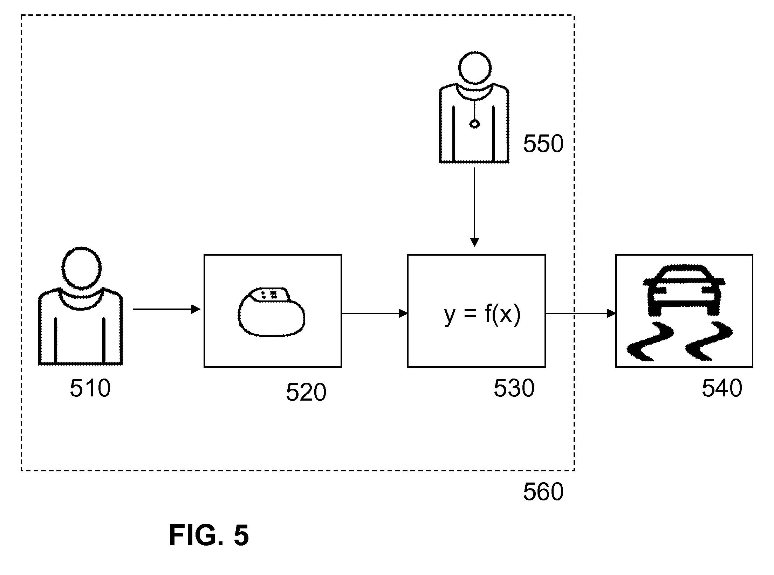

In der Figur 5 ist die erfindungsgemäße Lösung illustriert. Das elektronische Implantat 520 leitet zunächst einen oder mehrere den physiologischen Zustand beschreibende physiologische Daten von erfassten Parametern des Patienten 510 ab und signalisiert diese physiologischen Daten einer konfigurierbaren Klassifikationseinheit 530. Diese Klassifikationseinheit 530 kann z.B. von einem behandelnden Arzt 550 patientenindividuell derart konfiguriert werden, dass diese Klassifikationseinheit 530 einen Fahrtauglichkeitszustand eines Patienten beschreibenden Tauglichkeitsindex in Abhängigkeit der physiologischen Daten aus dem Implantat 520 bildet und an ein Fahrzeug bzw. Fahrassistenzsystem 540, d.h. eine Fahrzeugsteuerung signalisiert. Das hier eingesetzte Fahrassistenzsystem 540 bildet eine Maschinensteuerung im Sinne der Ansprüche und erhält von der Klassifikationseinheit im Unterschied zum Stand der Technik nicht einen direkten Fahrbefehl, sondern in Form des Tauglichkeitsindexes lediglich eine Information über den Fahrtauglichkeitszustand des Patienten. Das Fahrassistenzsystem 540 leitet dann, basierend auf der Fahrtauglichkeitsinformation und weiteren relevanten Fahrinformationen (Geschwindigkeit, Abstandssensoren, Gierrate, ...) eine angemessene Fahrbeeinflussung ab.

Grundsätzlich wird hier das Sicherheitssystem in ein Medizinproduktesystem 560 und das Fahrzeugsystem getrennt, so dass die Kompetenz für die Beschreibung und Klassifikation des Fahrtauglichkeitszustandes in der Kompetenz des Medizinprodukteherstellers und des Arztes ist, die Fahrzeugbeeinflussung selbst in der Kompetenz der Fahrzeughersteller und deren Zulieferer.

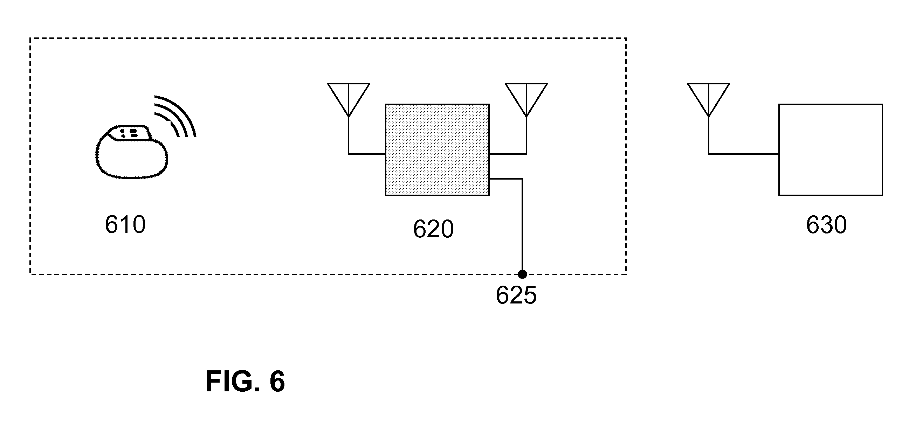

In der Figur 6 ist das Blockschaltbild des erfindungsgemäßen Sicherheitssystems dargestellt. Dieses beinhaltet ein elektronisches Implantat 610, das bevorzugt im MICS oder ISM-Band diagnostische oder therapeutische Informationen als physiologische Daten an eine Klassifikationseinheit 620 überträgt. Die Klassifikationseinheit 620 ist in diesem System Bestandteil eines Steuergerätes als Relaisstation zwischen Implantat und Fahrzeug, kann aber optional auch Bestandteil des elektronischen Implantates sein. Über eine dem Arzt zugängliche Schnittstelle 625 kann patientenindividuell die Zuordnung der Informationen des Implantates zu verschiedenen Fahrtauglichkeitsbeschreibungen erfolgen. Diese Fahrtauglichkeitsbeschreibung wird dann über eine im Fahrzeugsektor bevorzugte Schnittstelle an das Fahrzeugsystem 630 übertragen und kann dort als zusätzliche Information für Fahrassistenzsysteme genutzt werden.

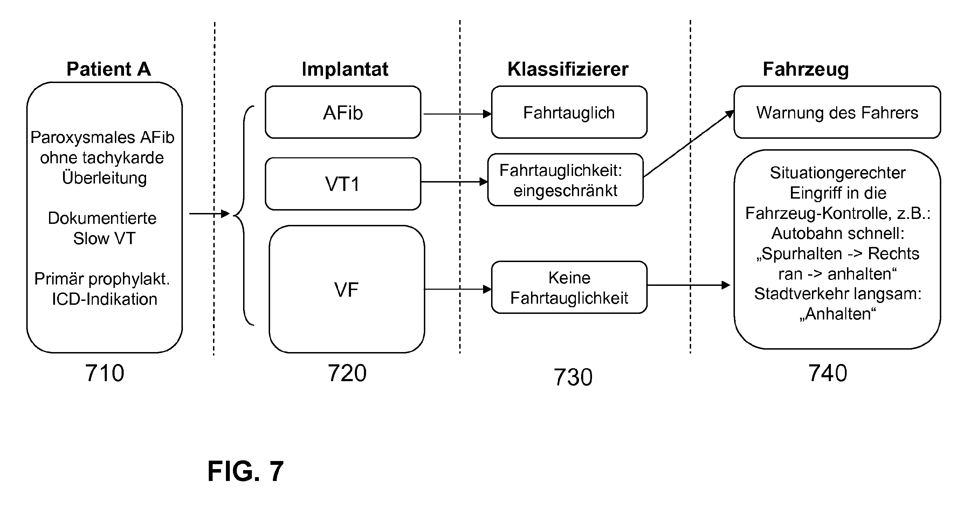

In der Figur 7 ist eine mögliche Klassifikation, individuell für einen Patienten A dargestellt. Dieser Patient A 710 hat einen Einkammer-ICD implantiert bekommen, da er die Indikationen für eine Primärprophylaxe erfüllt. Zusätzlich ist dem Arzt bekannt, dass der Patient an einem paroxysmalen Vorhofflimmern ohne tachykarde Überleitung und haemodynamische Konsequenzen leidet. Ebenso ist eine langsame ventrikuläre Tachykardie dokumentiert, die bei langer Dauer zu einem Schwindelgefühl des Patienten führen kann.

Das Implantat 720 - ein implantierbarer Kardioverter / Defibrillator (ICD) - kann die kardiologischen Zustände des Patienten klassifizieren und bei Bedarf automatisch therapieren. Aktiviert sind bei diesem Patienten eine Klasse zur Diagnostik des Vorhofflimmerns (AFib), die keine Therapie des ICD zu Folge hat, eine Klasse zu Detektion der typischen langsamen ventrikulären Tachykardie (VT1), die eine schmerzfreie ATP-Therapie einschließt und eine Klasse zu Erkennung ventrikulären Flimmerns (VF) mit einer automatischen Defibrillationsschocktherapie mit bis zu 8 Schocks in Folge.

In der Klassifikationseinheit 730 hat der betreuende Arzt die Fahrtauglichkeit des Patienten wie folgt hinterlegt: bei Vorhofflimmern ist die Fahrtauglichkeit nicht eingeschränkt. Bei einer langsamen ventrikulären Tachykardie ist die Fahrtauglichkeit eingeschränkt (d.h. mindestens für die folgenden 10 Minuten nach VT-Detektion noch gegeben) und in der ventrikulären Flimmerklasse ist die Fahrtauglichkeit nicht mehr gegeben.

Diese Information über die Fahrtauglichkeit wird an das Fahrassistenzsystem 740 weitergegeben, das Teil einer Maschinensteuerung bzw. Fahrzeugelektronik des Fahrzeugs ist. Im Falle der bedingten Fahrtauglichkeit weist das System den Fahrer an, das Fahrzeug umgehend abzustellen, greift aber nicht aktiv in den Fahrverlauf ein. Im Falle der nicht mehr vorhandenen Fahrtauglichkeit, übernimmt das Fahrassistenzsystem die Kontrolle über das Fahrzeug, mit dem Ziel, diese fahrsituationsbedingt sicher zum Stehen zu bringen. Allerdings werden dazu alle weiteren sensorischen Informationen des Fahrzeugs einbezogen, so dass die Fahrzeugreaktion der aktuellen Fahrsituation angepasst wird (z.B. Spurhalten und relativ langsames Abbremsen des Fahrzeuges bei schneller Autobahnfahrt vs. zügigem Anhaltens im langsamen Innenstadtverkehr).

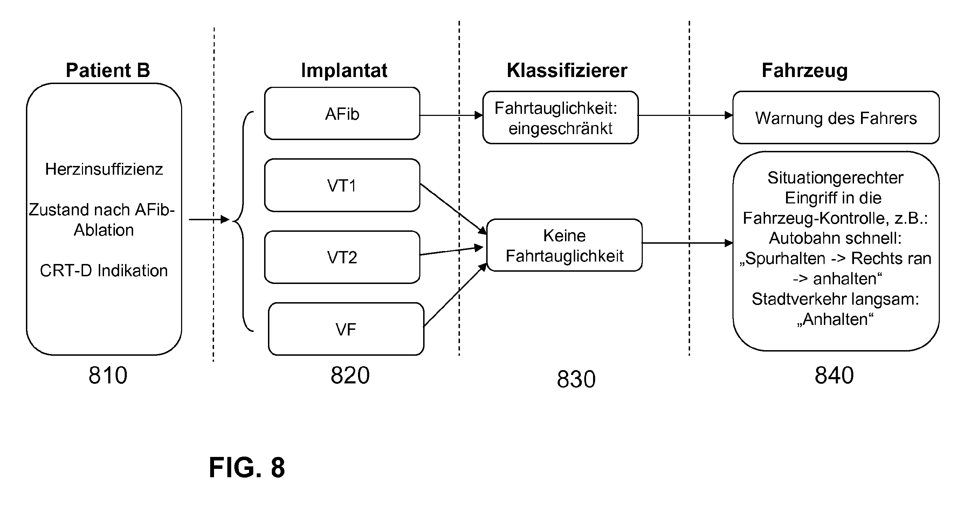

In Figur 8 ist eine mögliche Klassifikation, individuell für einen Patienten B dargestellt. Diese Patient B 810 hat einen Dreikammer-ICD (CRT-D) implantiert bekommen, da er die Indikationen für eine kardiale Resynchronisationstherapie auf Grund einer ausgeprägten Herzinsuffizienz erfüllt. Gleichzeitig wurde eine Vorhofflimmerablation durchgeführt, um ein tachykard übergeleitetes Vorhofflimmern zu therapieren.

Das Implantat 820 - ein ICD - kann die kardiologischen Zustände des Patienten klassifizieren und bei Bedarf automatisch therapieren. Aktiviert sind bei diesem Patienten eine Klasse zur Diagnostik des Vorhofflimmerns (AFib), die keine Therapie des ICD zu Folge hat, zwei Klassen zur Detektion ventrikulärer Tachykardien (VT1, VT2), die eine schmerzfreie ATP-Therapie und Schocktherapie einschließen und eine Klasse zu Erkennung ventrikulären Flimmerns (VF) mit einer automatischen Defibrillationsschocktherapie mit bis zu acht Schocks in Folge.

In der Klassifikationseinheit 830 hat der betreuende Arzt die Fahrtauglichkeit des Patienten wie folgt hinterlegt: Bei Vorhofflimmern ist die Fahrtauglichkeit bedingt eingeschränkt, da dieses zu einem Verlust der effektiven Resynchronisation führen kann und damit nach 15-20 Minuten Schwindelzustände oder kurze Bewusstseinsverlusten nicht ausgeschlossen werden können. In der ventrikulären Flimmerklasse ist die Fahrtauglichkeit nicht mehr gegeben.

Diese Information über die Fahrtauglichkeit wird an das Fahrassistenzsystem 840 weitergegeben. Im Falle der bedingten Fahrtauglichkeit weist das System den Fahrer an, das Fahrzeug umgehend abzustellen, greift aber nicht aktiv in den Fahrverlauf ein. Im Falle der nicht mehr vorhandenen Fahrtauglichkeit, übernimmt das Fahrassistenzsystem die Kontrolle über das Fahrzeug, mit dem Ziel, diese fahrsituationsbedingt sicher zum Stehen zu bringen. Allerdings werden dazu alle weiteren sensorischen Informationen des Fahrzeugs einbezogen, so dass die Fahrzeugreaktion der aktuellen Fahrsituation angepasst wird (z.B. Spurhalten und relativ langsames Abbremsen des Fahrzeuges bei schneller Autobahnfahrt vs. zügigem Anhaltens im langsamen Innenstadtverkehr).

### Bezugszeichenliste

- 110: Implantat
- 120: Fahrzeug
- 210: elektronisches Implantat
- 220: Fahrzeugschlüssel
- 230: Fahrzeug
- 320: MICS-Band-Radio
- 330: Antenne
- 350: Antenne
- 340: Kommunikationsmodul
- 360: Sicherheitsserver
- 370: Wartungsschnittstelle
- 410: Patient
- 420: Implantat
- 430: Fahrzeug
- 510: Patient
- 520: Implantat
- 530: Klassifikationseinheit
- 540: Fahrassistenzsystem
- 550: Arzt
- 560: Medizinproduktesystem
- 610: Implantat
- 620: Klassifikationseinheit
- 625: Schnittstelle
- 630: Fahrzeugsystem
- 710: Patient A
- 720: Implantat
- 730: Klassifikationseinheit
- 740: Fahrassistenzsystem
- 810: Patient B
- 820: Implantat
- 830: Klassifikationseinheit
- 840: Fahrzeug

## Patentansprüche

1. Sicherheitssystem umfassend ein Implantat, ein Steuergerät und eine Maschine,
von denen die Maschine eine Steuereinheit aufweist, die ausgebildet ist,
- einen Betrieb der Maschine nur im Falle des Empfangs einer eindeutigen Kennung zu ermöglichen
- und eine Betriebsart der Maschine in Abhängigkeit von einem empfangenen Tauglichkeitsindex einzustellen, der die Tauglichkeit eines Implantatträgers, eine Maschine zu bedienen, beschreibt,
von denen das Implantat ausgebildet ist, physiologische Daten eines Implantatträgers zu erfassen
und von denen das Steuergerät
- eine erste Schnittstelle umfasst, die für eine drahtlose Kommunikation mit dem Implantat ausgebildet ist,
- eine zweite Schnittstelle umfasst, die für eine drahtlose Kommunikation mit der Steuereinheit der Maschine ausgebildet ist,
- und einen Speicher für eine eindeutige Kennung umfasst,
wobei die Maschine ein Fahrzeug ist und die Steuereinheit ein Teil einer Fahrzeugelektronik,
und entweder
(a.1) wobei das Implantat ausgebildet ist, aus erfassten physiologischen Daten einen Tauglichkeitsindex zu generieren und den Tauglichkeitsindex an das Steuergerät zu übertragen, und
(a.2) wobei die erste Schnittstelle ausgebildet ist, den Tauglichkeitsindex zu empfangen, und wobei die zweite Schnittstelle ausgebildet ist, den Tauglichkeitsindex zusammen mit der eindeutigen Kennung an die Steuereinheit der Maschine zu übertragen,
oder
(b.1) wobei das Implantat ausgebildet ist, die physiologischen Daten selbst an das Steuergerät zu übertragen, und
(b.2) wobei die erste Schnittstelle ausgebildet ist, vom Implantat die physiologischen Parameter zu empfangen, und wobei die zweite Schnittstelle ausgebildet ist, den Tauglichkeitsindex zusammen mit der eindeutigen Kennung an die Steuereinheit der Maschine zu übertragen, wobei das Steuergerät eine Klassifikationseinheit aufweist, die ausgebildet ist, die physiologischen Daten zu empfangen, jeweils empfangene physiologische Daten einer von mehreren vorgegebenen Tauglichkeitsklassen zuzuordnen und einen die jeweils zugeordnete Tauglichkeitsklasse bezeichnenden Index zu generieren und als Tauglichkeitsindex der zweiten Schnittstelle zur Verfügung zu stellen.

2. Sicherheitssystem nach Anspruch 1, **dadurch gekennzeichnet, dass** die zweite Schnittstelle eine kurze Reichweite von wenigen Zentimetern hat.

3. Sicherheitssystem nach einem der Ansprüche 1 oder 2, **dadurch gekennzeichnet, dass** die zweite Schnittstelle ausgebildet ist, Daten im ISM-Frequenzband zu übertragen.

4. Sicherheitssystem nach einem der Ansprüche 1 bis 3, **dadurch gekennzeichnet, dass** das Steuergerät als elektronischer Fahrzeugschlüssel ausgebildet ist.

5. Sicherheitssystem nach einem der Ansprüche 1 bis 4, **dadurch gekennzeichnet, dass** das Implantat ein Herzstimulator ist.

## Claims

1. A security system comprising an implant, a control device, and a machine,
wherein the machine has a control device which is designed to
- permit operation of the machine only in the event that a unique identifier is received, and
- set an operating mode of the machine depending on a suitability index which is received, the suitability index describing the suitability of an implant wearer to operate a machine,
wherein the implant is designed to detect physiological data on an implant wearer,
and wherein the control device has
- a first interface, which is designed for wireless communication with the implant,
- a second interface, which is designed for wireless communication with the control unit of the machine,
- and a memory for a unique identifier,
wherein the machine is a vehicle and the control unit is part of a vehicle electronics system,
and either
(a.1) wherein the implant is designed to generate a suitability index from detected physiological data and transmit the suitability index to the control device, and
(a.2) wherein the first interface is designed to receive the suitability index, and wherein the second interface is designed to transfer the suitability index together with the unique identifier to the control unit of the machine,
or
(b.1) wherein the implant is designed to transmit the physiological data itself to the control device, and
(b.2) wherein the first interface is designed to receive the physiological parameters from the implant, and wherein the second interface is designed to transmit the suitability index together with the unique identifier to the control unit of the machine, wherein the control device has a classification unit which is designed to receive the physiological data, assign received physiological data to one of several predefined suitability classes, and generate an index which characterizes the particular assigned suitability class, and to provide the index, as a suitability index, to the second interface.

2. The security system according to claim 1, **characterized in that** the second interface has a short range of a few centimeters.

3. The security system according to any one of claims 1 or 2, **characterized in that** the second interface is designed to transmit data in the ISM frequency band.

4. The security system according to any one of claims 1 to 3, **characterized in that** the control device is designed as an electronic vehicle key.

5. The security system according to any one of claims 1 to 4, **characterized in that** the implant is a cardiac stimulator.

## Revendications

1. Système de sécurité comprenant un implant, un appareil de commande et une machine,
parmi lesquels la machine présente une unité de commande qui est conçue pour
- permettre un fonctionnement de la machine uniquement en cas de réception d'une identification explicite
- et pour régler un mode de fonctionnement de la machine en fonction d'un indice d'aptitude reçu, qui décrit l'aptitude d'un porteur d'implant à se servir d'une machine,
parmi lesquels l'implant est conçu pour saisir des données physiologiques d'un porteur d'implant,
et parmi lesquels l'appareil de commande
- comprend une première interface qui est conçue pour une communication sans fil avec l'implant,
- comprend une deuxième interface qui est conçue pour une communication sans fil avec l'unité de commande de la machine,
- et comprend une mémoire pour une identification explicite,
où la machine est un véhicule et l'unité de commande est une partie d'une électronique de véhicule,
et, soit
(a.1) où l'implant est conçu pour générer un indice d'aptitude à partir des données physiologiques saisies et à transmettre l'indice d'aptitude à l'appareil de commande, et
(a.2) où la première interface est conçue pour recevoir l'indice d'aptitude et où la deuxième interface est conçue pour transmettre l'indice d'aptitude, ensemble avec l'identification explicite, à l'unité de commande de la machine,
soit
(b.1) où l'implant est conçu pour transmettre lui-même les données physiologiques à l'appareil de commande, et
(b.2) où la première interface est conçue pour recevoir les paramètres physiologiques de l'implant, et où la deuxième interface est conçue pour transmettre l'indice d'aptitude, ensemble avec l'identification explicite, à l'unité de commande de la machine, où l'appareil de commande présente une unité de classification qui est conçue pour recevoir les données physiologiques, classer les données physiologiques reçues respectivement dans l'une des plusieurs classes d'aptitude prédéterminées et pour générer un indice désignant la classe d'aptitude respectivement associée et pour le mettre à disposition en tant qu'indice d'aptitude de la deuxième interface.

2. Système de sécurité selon la revendication 1, **caractérisé en ce que** la deuxième interface possède une portée courte de quelques centimètres.

3. Système de sécurité selon l'une des revendications 1 ou 2, **caractérisé en ce que** la deuxième interface est conçue pour transmettre des données dans la bande des fréquences ISM.

4. Système de sécurité selon l'une des revendications 1 à 3, **caractérisé en ce que** l'appareil de commande est conçu sous forme d'une clé électronique de véhicule.

5. Système de sécurité selon l'une des revendications 1 à 4, **caractérisé en ce que** l'implant est un stimulateur cardiaque.
